# EUROPEAN PATENT SPECIFICATION

(11) **EP 3 932 325 B1**
(45) Date of publication and mention of the grant of the patent: **12.02.2025**
(21) Application number: 20784417.6
(22) Date of filing: 23.03.2020
(51) Int. Cl.: A61B 8/12, A61B 1/00, A61B 1/07, A61B 8/00

(54) **IMAGE DIAGNOSIS CATHETER**
BILDDIAGNOSEKATHETER
CATHÉTER DE DIAGNOSTIC D'IMAGE

(30) Priority: 29.03.2019 JP 2019068660
(43) Date of publication of application: 05.01.2022
(73) Proprietor: TERUMO Kabushiki Kaisha, Tokyo 151-0072 (JP)
(72) Inventor: SAKAGUCHI Yuki, Ashigarakami-gun, Kanagawa 259-0151 (JP)
(74) Representative: Casalonga
(86) International application number: PCT/JP2020/012777
(87) International publication number: WO 2020/203423

(56) References cited:
- WO-A2-00/25297
- JP-A- 2002 005 822
- JP-A- 2015 515 918
- JP-A- 2017 153 621
- US-A1- 2013 331 820
- US-A1- 2014 257 102
- US-A1- 2017 127 920

## Description

### Technical Field

The present disclosure relates to a diagnostic imaging catheter.

### Background Art

WO 00/25297 relates to an ultrasound imaging assembly including a housing having a longitudinal axis, a proximal end, and a distal ending defining a receptacle.

US 2014/0257102 A1 relates to a system for imaging a vessel of a patient comprising an elongated sheath with proximal and distal ends.

US 2017/0127920 A1 relates to a fixing sleeve that is fitted outward on a connecting portion between a connecting pipe and a treatment tool inserting tube for fixation of the connecting portion comprises a cylindrical sleeve body, an arc-shaped portion, and a projection portion.

US 2013/0331820 A1 relates to a catheter comprising a sheath to be inserted into a living body, including a tubular reinforcement layer of at least one layer, which is formed with a spiral slit, a termination end of the spiral slit, and a slit termination portion.

In the related art, as an example of a diagnostic imaging catheter that obtains a tomographic image of a blood vessel or the like, a catheter that obtains an image by an intra vascular ultra sound (IVUS) has been known. PTL 1 discloses a diagnostic imaging catheter of this type. PTL 1 describes an ultrasound transducer as a signal transmission and reception member, which is mounted on a terminal housing as a housing.

### Citation List

### Patent Literature

PTL 1: JP-A-2006-198425

### Summary of Invention

### Technical Problem

The housing on which the signal transmission and reception member is mounted is made of a hard and hard-to-deform material in order to stabilize a rotation axis of the transmission and reception member with respect to rotation of a drive shaft. On the other hand, the housing is required to have followability for easily moving forward along a bent blood vessel shape.

An object of the present disclosure is to provide a diagnostic imaging catheter including a housing capable of improving followability to a blood vessel shape.

### Solution to Problem

A diagnostic imaging catheter according to a first aspect of the present disclosure includes: a diagnostic imaging catheter according to claim 1. Preferred embodiments are disclosed in the dependent claims.

As one embodiment of the present disclosure, a side surface of the housing includes a support surface that supports the transmission and reception member, and in an upper side view of the housing as viewed from a support surface side, the inclined portion is formed on the side surface of the housing located on at least one side of the transmission and reception member.

As one embodiment of the present disclosure, a side surface of the housing includes a support surface that supports the transmission and reception member, and the inclined portion is formed at a position of the side surface of the housing on a back side of the support surface.

As one embodiment of the present disclosure, a back side of the support surface of the side surface of the housing is defined by a peripheral surface.

As one embodiment of the present disclosure, a proximal protrusion portion protruding toward an inner surface of the sheath from the transmission and reception member supported by the support surface is provided on a proximal side of the side surface of the housing with respect to the support surface.

As one embodiment of the present disclosure, a distal protrusion portion protruding toward an inner surface of the sheath from the transmission and reception member supported by the support surface is provided on a distal side of the side surface of the housing with respect to the support surface.

As one embodiment of the present disclosure, an angle of the inclined portion with respect to the central axis line increases toward the distal side.

The transmission and reception member is an ultrasound transducer capable of transmitting and receiving ultrasound on a ultrasound transmission and reception surface, and a distal end surface of the ultrasound transducer is formed of a convex curved surface.

### Advantageous Effect of Invention

According to the present disclosure, it is possible to provide a diagnostic imaging catheter including a housing capable of improving followability to a blood vessel shape.

### Brief Description of Drawings

[Fig. 1] Fig. 1 is a diagram showing a state in which a diagnostic imaging catheter as an embodiment of the present disclosure and a diagnostic imaging apparatus are connected.
[Fig. 2] Fig. 2 is a cross-sectional view showing a cross section parallel to a longitudinal direction at a distal end portion of the diagnostic imaging catheter shown in Fig. 1.
[Fig. 3] Fig. 3 is a horizontal side view of a vicinity of an imaging core portion of the diagnostic imaging catheter shown in Fig. 1.
[Fig. 4] Fig. 4 is an upper side view of the vicinity of the imaging core portion of the diagnostic imaging catheter shown in Fig. 1.
[Fig. 5] Fig. 5 is a perspective view of the vicinity of the imaging core portion of the diagnostic imaging catheter shown in Fig. 1.
[Fig. 6] Fig. 6 is an in-use state diagram showing a state in which the diagnostic imaging catheter shown in Fig. 1 is inserted into a blood vessel.
[Fig. 7] Fig. 7 is a diagram showing a specific example of an ultrasound transducer in which an outer shape of an ultrasound transmission and reception surface is an elliptical shape.

### Description of Embodiments

Hereinafter, an embodiment of a diagnostic imaging catheter according to the present disclosure will be described with reference to the drawings. In the drawings, common members and portions are denoted by the same reference numerals. Hereinafter, for convenience of description, in the diagnostic imaging catheter according to the present disclosure, a longitudinal direction of the diagnostic imaging catheter is referred to as a "longitudinal direction A". In addition, in the longitudinal direction A of the diagnostic imaging catheter, a side on which the diagnostic imaging catheter is inserted into a living body is referred to as a "distal side", and a side opposite thereto is referred to as a "proximal side". In addition, a direction from the proximal side toward the distal side of the diagnostic imaging catheter according to the present disclosure may be simply referred to as an "insertion direction A1". In addition, a direction from the distal side to the proximal side of a diagnostic imaging catheter 1 may be simply referred to as a "removal direction A2".

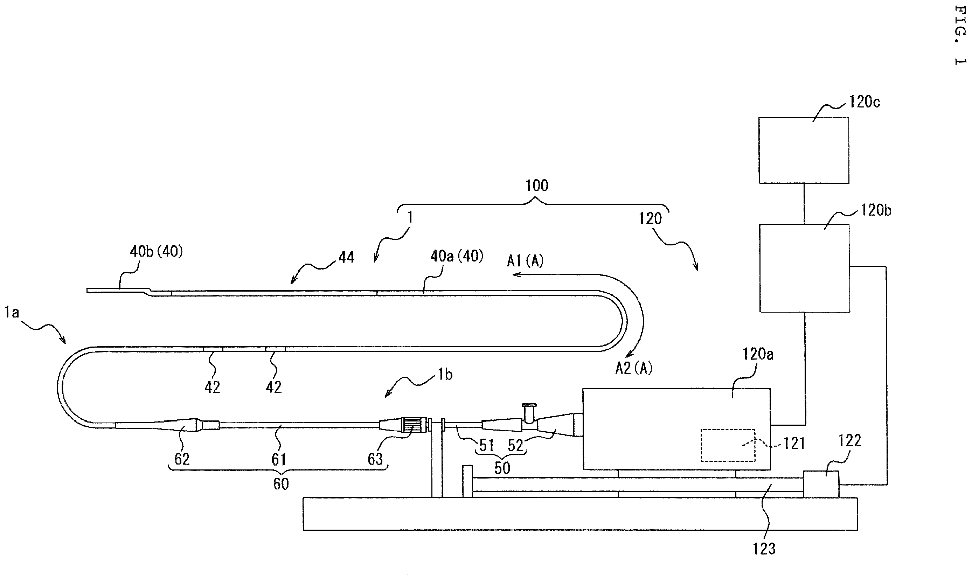

First, an example of a diagnostic imaging system to which the diagnostic imaging catheter according to the present disclosure can be applied will be described. Fig. 1 is a diagram showing a diagnostic imaging system 100 including the diagnostic imaging catheter 1 as an embodiment of the present disclosure.

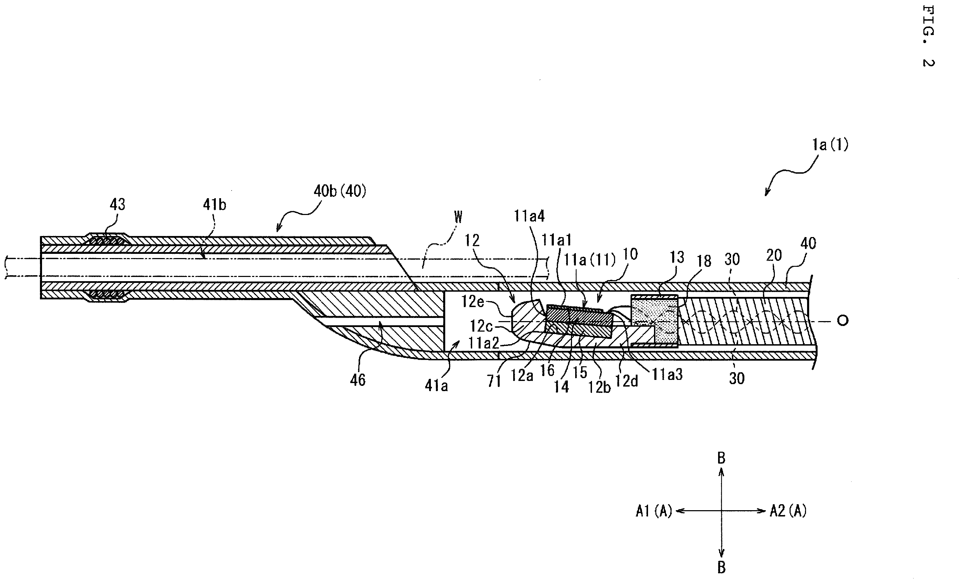

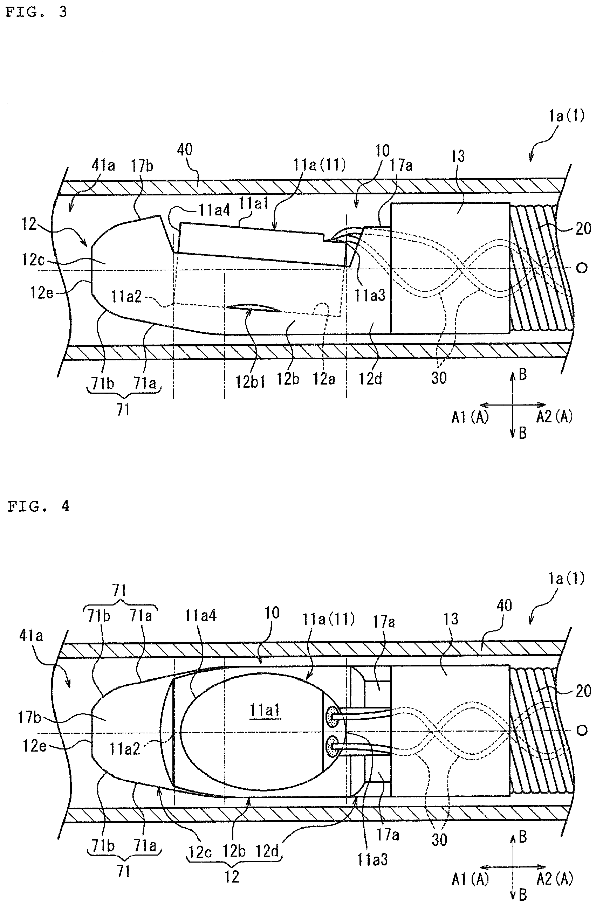

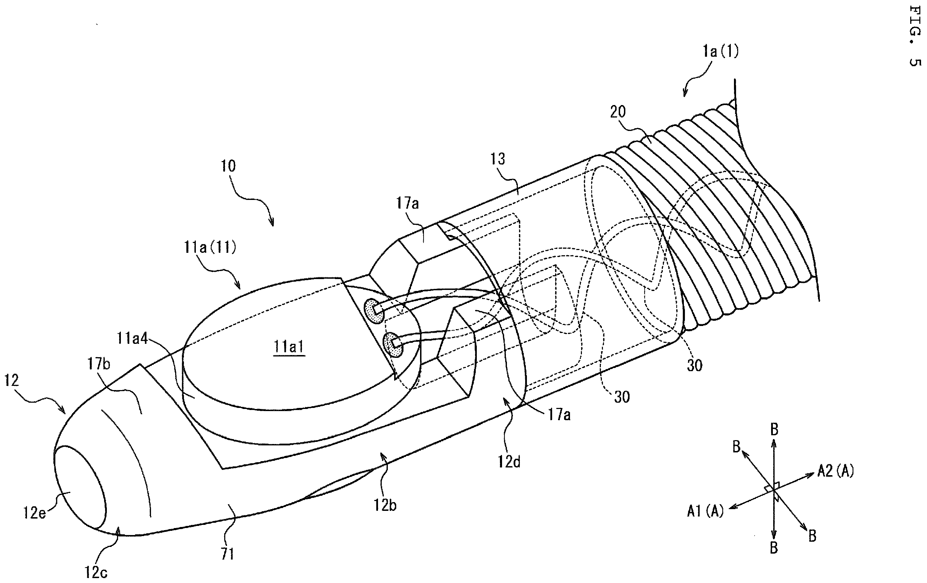

The diagnostic imaging system 100 includes the diagnostic imaging catheter 1 and a diagnostic imaging apparatus 120. Fig. 1 shows a state in which the diagnostic imaging catheter 1 is connected to the diagnostic imaging apparatus 120. Fig. 2 is a cross-sectional view showing a cross section parallel to the longitudinal direction A at a distal end portion that is an end portion on the distal side of the diagnostic imaging catheter 1. Fig. 3 is an upper side view of a vicinity of an imaging core portion 10 of the diagnostic imaging catheter 1. Fig. 4 is a horizontal side view of the vicinity of the imaging core portion 10 of the diagnostic imaging catheter 1. Fig. 5 is a perspective view of the vicinity of the imaging core portion 10 of the diagnostic imaging catheter 1. In Fig. 5, a sheath 40 is omitted.

### <Diagnostic Imaging Catheter 1>

The diagnostic imaging catheter 1 of the present embodiment can be applied to IVUS. As shown in Fig. 1, the diagnostic imaging catheter 1 is driven by being connected to the diagnostic imaging apparatus 120. More specifically, the diagnostic imaging catheter 1 of the present embodiment is connected to a drive unit 120a of the diagnostic imaging apparatus 120.

As shown in Fig. 1, the diagnostic imaging catheter 1 includes an insertion portion 1a and an operation portion 1b. The insertion portion 1a is a portion of the diagnostic imaging catheter 1 that is inserted into the living body and used. The operation portion 1b is a portion of the diagnostic imaging catheter 1 that is operated outside the living body in a state where the insertion portion 1a is inserted into the living body. In the diagnostic imaging catheter 1 of the present embodiment, a portion on the distal side of a distal side connector 62 (see Fig. 1) described later is the insertion portion 1a, and a portion on the proximal side from the distal side connector 62 is the operation portion 1b.

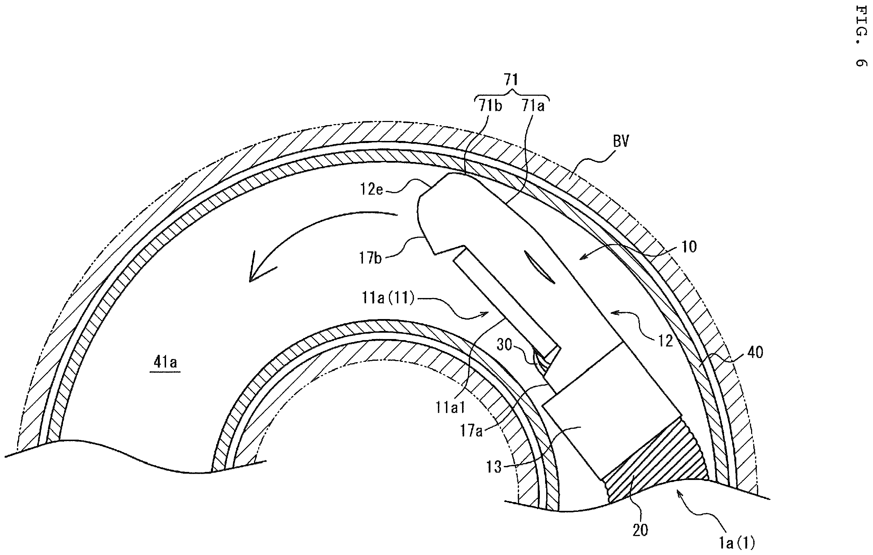

As shown in Figs. 1 and 2, the insertion portion 1a includes the imaging core portion 10, a drive shaft 20, a signal line 30, and a sheath 40. The imaging core portion 10 is coupled to the distal side of the drive shaft 20. The sheath 40 is inserted into the living body and used (see Fig. 6). The imaging core portion 10, the drive shaft 20, and the signal line 30 are located in the sheath 40 and are inserted into the living body together with the sheath 40 and used (see Fig. 6).

As shown in Fig. 1, the operation portion 1b includes an inner tube member 50 and an outer tube member 60. The inner tube member 50 holds an end portion on the proximal side of the drive shaft 20. The outer tube member 60 holds an end portion on the proximal side of the sheath 40. When the inner tube member 50 moves in a central axis direction in the outer tube member 60, the imaging core portion 10, the drive shaft 20, and the signal line 30 shown in Fig. 2 can move in the sheath 40 in the longitudinal direction A. The drive shaft 20 and the signal line 30 extend not only in a region of the insertion portion 1a but also in a region of the operation portion 1b in the longitudinal direction A through the inside of the inner tube member 50 and the outer tube member 60.

### [Imaging Core Portion 10]

As shown in Fig. 2, the imaging core portion 10 is located in the sheath 40 inserted into the living body. The imaging core portion 10 of the present embodiment includes a transmission and reception member 11 capable of transmitting and receiving a signal, a housing 12 holding the transmission and reception member 11, and a contrast marker 13.

The transmission and reception member 11 of the present embodiment is an ultrasound transducer 11a capable of transmitting and receiving an ultrasonic signal. Hereinafter, the ultrasound transducer 11a as the transmission and reception member 11 will be described as an example, but the transmission and reception member 11 is not limited to the ultrasound transducer 11a, and may be, for example, an optical element capable of transmitting and receiving an optical signal. An example of the optical element capable of transmitting and receiving the optical signal includes a ball lens that is provided at a distal end of an optical fiber and has a lens function of condensing light and a reflection function of reflecting light.

The ultrasound transducer 11a as the transmission and reception member 11 of the present embodiment includes a piezoelectric element 14, a support member 15, and an acoustic matching member 16.

Specifically, the piezoelectric element 14 includes a flat piezoelectric body, a first electrode laminated on at least one side in a thickness direction of the piezoelectric body, and a second electrode laminated on at least the other side in the thickness direction of the piezoelectric body. Hereinafter, for convenience of description, a side on which an ultrasound transmission and reception surface 11a1 capable of transmitting and receiving ultrasound of the ultrasound transducer 11a is located in the thickness direction of the piezoelectric body is referred to as a "front surface side", and a side opposite to the ultrasound transmission and reception surface 11a1 of the ultrasound transducer 11a in the thickness direction of the piezoelectric body is referred to as a "back surface side".

The piezoelectric body of the piezoelectric element 14 is formed of, for example, a piezoelectric ceramic sheet. An example of a material of the piezoelectric ceramic sheet includes a piezoelectric ceramic material such as lead zirconate titanate (PZT) and lithium niobate. The piezoelectric body may be formed of crystal instead of the piezoelectric ceramic material.

The first electrode and the second electrode of the piezoelectric element 14 can be formed, for example, by laminating electrode layers on both sides in the thickness direction of the piezoelectric body by an ion plating method, a vapor deposition method, or a sputtering method using a mask material. Examples of a material of the first electrode and the second electrode include metals such as silver, chromium, copper, nickel, and gold, and laminates of these metals.

The first electrode is laminated only on the front surface side of the piezoelectric body. The second electrode is laminated on the back surface side of the piezoelectric body, and a part of the second electrode is folded back to the front surface side of the piezoelectric body. That is, the second electrode of the present embodiment is a folded-back electrode. However, the first electrode and the second electrode may not be folded-back electrodes. In addition, the second electrode may not be a folded-back electrode, and the first electrode may be a folded-back electrode in which a part of the first electrode is folded back to the back surface side to form the second electrode.

The support member 15 supports the piezoelectric element 14 from the back surface side of the piezoelectric element. Specifically, the support member 15 is laminated on the piezoelectric element 14 to cover an entire region of the back surface side of the piezoelectric element 14. Thus, the ultrasound from the piezoelectric element 14 that is noise can be absorbed. That is, the support member 15 of the present embodiment is a sound absorbing layer that absorbs the ultrasound of the piezoelectric element 14.

The sound absorbing layer as the support member 15 can be formed by a method of bonding a sheet material forming the sound absorbing layer to the piezoelectric element 14, a method of applying and curing a liquid sound absorbing material forming the sound absorbing layer, or the like. An example of a material of the support member 15 includes an epoxy resin in which rubber and a metal powder such as tungsten powder are dispersed.

The acoustic matching member 16 is laminated to cover the front surface side of the piezoelectric element 14. More specifically, the acoustic matching member 16 of the present embodiment is laminated to cover the entire region of the front surface side of the piezoelectric element 14 except for a portion of the piezoelectric element 14 to which the signal line 30 is connected to the first electrode and the second electrode. By providing the acoustic matching member 16, propagation efficiency of the ultrasound to an object can be enhanced. That is, the acoustic matching member 16 of the present embodiment is an acoustic matching layer that enhances the propagation efficiency of the ultrasound.

The acoustic matching layer as the acoustic matching member 16 can be formed by a method of bonding a sheet material forming the acoustic matching layer to the piezoelectric element 14, a method of applying and curing a liquid acoustic matching material forming the acoustic matching layer, or the like. An example of the material of the acoustic matching member 16 includes a resin material such as the epoxy resin. Further, the acoustic matching member 16 may be a laminated body of resin layers made of the resin material.

The ultrasound transmission and reception surface 11a1 of the ultrasound transducer 11a as the transmission and reception member 11 of the present embodiment is a surface of the ultrasound transducer 11a. That is, in the ultrasound transducer 11a of the present embodiment, the planar ultrasound transmission and reception surface 11a1 is defined by the acoustic matching member 16.

Further, the ultrasound transducer 11a as the transmission and reception member 11 of the present embodiment has an elliptical outer shape in a plan view as viewed in the thickness direction of the piezoelectric body, that is, in a plan view of the ultrasound transmission and reception surface 11a1. Details thereof will be described later.

As shown in Figs. 2 to 4, the housing 12 holds the ultrasound transducer 11a as the transmission and reception member 11 in the sheath 40. The proximal side of the housing 12 is connected to the drive shaft 20. As shown in Figs. 2 to 4, a side surface of the housing 12 that faces a radial direction B of the sheath 40 includes an inclined portion 71 that is inclined to approach a central axis line O of the drive shaft 20 toward the distal side until reaching the distal end. The "side surface of the housing" is a surface of the housing that faces the radial direction B of the sheath, and means a surface that constitutes an entire region around the sheath in the radial direction B. The side surface of the housing 12 of the present embodiment includes a support surface 12a that supports the ultrasound transducer 11a serving as the transmission and reception member 11. The side surface of the housing 12 will be described in detail later.

The housing 12 of the present embodiment includes a main body portion 12b, a distal end portion 12c, and a proximal end portion 12d. The main body portion 12b includes the above support surface 12a. The distal end portion 12c is located on the distal side of the main body portion 12b and includes a distal end. The proximal end portion 12d is located on the proximal side of the main body portion 12b, and is connected to the contrast marker 13. Each portion of the housing 12 will be described in detail later.

The housing 12 can be made of, for example, a resin such as polycarbonate. The housing 12 is formed by, for example, injection molding of the resin material. The housing 12 may be made of metal. Such a housing 12 can be made of, for example, stainless steel, gold-plated stainless steel, a platinum iridium alloy, and a platinum zirconia alloy. In addition, such a housing 12 is formed by cutting from a metal ingot, metallic powder injection molding (MIM), or the like. Further, the housing 12 may be made of ceramics prepared by firing zirconia or the like.

The contrast marker 13 has a substantially cylindrical outer shape, and is connected to the proximal end portion 12d of the housing 12 at the distal side. In the present embodiment, in a state where the proximal end portion 12d of the housing 12 is inserted into the contrast marker 13, the housing 12 and the contrast marker 13 are bonded to each other by an adhesive or the like. However, a connection configuration between the housing 12 and the contrast marker 13 is not limited to the above configuration. The contrast marker 13 can be, for example, a metal coil or a metal pipe having high X-ray impermeableness such as platinum, gold, iridium, and tungsten.

As shown in Fig. 2, for example, an absorbing member 18 formed of the same material as that of the support member 15 described above is disposed inside the substantially cylindrical contrast marker 13 of the present embodiment. Since the inside of the contrast marker 13 is filled with such an absorbing member 18, it is possible to prevent the ultrasound from being transmitted from the ultrasound transducer 11a serving as the transmission and reception member 11 toward a portion different from a target portion. Further, it is possible to prevent the ultrasound transducer 11a serving as the transmission and reception member 11 from receiving the ultrasound reflected at a portion different from the target portion.

The imaging core portion 10 of the present embodiment includes the contrast marker 13 on the proximal side of the housing 12, and may also be an imaging core portion without the contrast marker 13. That is, the proximal end portion 12d of the housing 12 may be connected to the drive shaft 20, which will be described later, without the contrast marker 13. When the imaging core portion without the contrast marker 13 is formed in this way, the housing 12 itself may be formed of a material having a contrast property, such as metal, a resin containing a material having high X-ray impermeableness, or ceramics.

### [Drive Shaft 20]

The drive shaft 20 is rotatable around the central axis line O in the sheath 40. The imaging core portion 10 described above is attached to the drive shaft 20 in the sheath 40. Therefore, the drive shaft 20 rotates the imaging core portion 10 around the central axis line O in the sheath 40. More specifically, the drive shaft 20 rotates around the central axis line O in the sheath to rotate the transmission and reception member 11, the housing 12, and the contrast marker 13 connected thereto around the central axis line O. A power source for rotating the drive shaft 20 is a motor 121 (see Fig. 1) of the diagnostic imaging apparatus 120 to be described later.

The drive shaft 20 is formed of a tubular body having flexibility. The signal line 30 connected to the ultrasound transducer 11a as the transmission and reception member 11 is disposed inside the drive shaft 20. The drive shaft 20 is, for example, a multi-layer coil having different winding directions around an axis. Examples of a material of the coil include the stainless steel and a nickeltitanium (Ni-Ti) alloy. By providing such a drive shaft 20, even if two electric signal lines are formed of a double spiral twisted pair cable as the signal line 30, the shielding property can be improved and an influence of noise generated from the electric signal line can be reduced.

The drive shaft 20 extends through the inside of the inner tube member 50 and the outer tube member 60 to a hub 52 to be described later. The hub 52 is located at the proximal end portion of the inner tube member 50. That is, the drive shaft 20 extends from the distal end portion of the insertion portion 1a to the proximal end portion of the operation portion 1b in the longitudinal direction A.

### [Signal Line 30]

The signal line 30 extends into the drive shaft 20, and electrically or optically connects the transmission and reception member 11 and the diagnostic imaging apparatus 120. The signal line 30 of the present embodiment is an electric signal line that electrically connects the ultrasound transducer 11a serving as the transmission and reception member 11 and the diagnostic imaging apparatus 120. Similarly to the drive shaft 20, the electric signal line as the signal line 30 of the present embodiment extends from the distal end portion of the insertion portion 1a to the proximal end portion of the operation portion 1b in the longitudinal direction A. A plurality of (two in the present embodiment) electric signal lines are provided as the signal line 30 of the present embodiment, and are respectively connected to the first electrode and the second electrode of the ultrasound transducer 11a described above. The plurality of electric signal lines as the signal line 30 are, for example, a twisted pair cable in which the two electric signal lines are twisted.

The signal line 30 of the present embodiment is an electric signal line. Alternatively, in a case of a configuration in which the transmission and reception member 11 can transmit and receive an optical signal, the signal line 30 may be, for example, an optical fiber line.

### [Sheath 40]

As shown in Fig. 2, the sheath 40 defines a first hollow portion 41a and a second hollow portion 41b. The imaging core portion 10, the drive shaft 20, and the signal line 30 are accommodated in the first hollow portion 41a. The imaging core portion 10, the drive shaft 20, and the signal line 30 can move forward and backward in the longitudinal direction A in the first hollow portion 41a. A guide wire W can be inserted into the second hollow portion 41b. In the present embodiment, a tubular guide wire insertion portion 40b that defines the second hollow portion 41b is located to be substantially parallel to the distal end portion of a tubular main body portion 40a that defines the first hollow portion 41a. The main body portion 40a and the guide wire insertion portion 40b can be formed by joining different tube members by thermal fusion or the like, and the present disclosure is not limited to such a forming method.

The main body portion 40a is provided with a marker portion 42 that has X-ray contrast properties and is formed of a material impermeable to X-rays. The guide wire insertion portion 40b is also provided with a marker portion 43 having X-ray contrast properties. The marker portions 42 and 43 can be, for example, a metal coil or a metal pipe having high X-ray impermeableness such as platinum, gold, iridium, and tungsten.

In a range in which the ultrasound transducer 11a as the transmission and reception member 11 moves in the longitudinal direction A of the sheath 40, a window portion 44 in which ultrasound permeability is higher than that at other portions is provided. More specifically, the window portion 44 of the present embodiment is provided in the main body portion 40a of the sheath 40.

The window portion 44 of the main body portion 40a and the guide wire insertion portion 40b are made of a material having flexibility, and the material is not particularly limited. Examples of the material include various thermoplastic elastomers such as polyethylene, styrene, polyolefin, polyurethane, polyester, polyamide, polyimide, polybutadiene, trans-polyisoprene, fluorine rubber, and chlorinated polyethylene, and polymer alloys, polymer blends, laminates, and the like that combine one or more of these substances can also be used.

The proximal side of the window portion 44 of the main body portion 40a includes a reinforced portion reinforced by a material having higher rigidity than the window portion 44. The reinforced portion is formed by, for example, disposing a reinforcing member, in which a metal wire made of the stainless steel or the like is braided in a mesh shape, in a tubular member having flexibility such as a resin. The tubular member may be formed of the same material as that of the window portion 44.

It is preferable to dispose a hydrophilic lubricating coating layer that exhibits lubricity when wet on an outer surface of the sheath 40.

A communication hole 46 that communicates the inside with the outside of the first hollow portion 41a is formed in the distal end portion of the main body portion 40a of the sheath 40. During priming, gas in the main body portion 40a can be discharged through the communication hole 46.

### [Inner Tube Member 50 and Outer Tube Member 60]

As shown in Fig. 1, the inner tube member 50 includes an inner tube 51 and the hub 52. The inner tube 51 is inserted into the outer tube member 60 to be movable forward and backward. The hub 52 is provided on the proximal side of the inner tube 51.

As shown in Fig. 1, the outer tube member 60 includes an outer tube 61, the distal side connector 62, and a proximal side connector 63. The outer tube 61 is located on an outer side in the radial direction (the same direction as the radial direction B of the sheath 40) of the inner tube 51, and the inner tube 51 moves forward and backward in the outer tube 61. The distal side connector 62 connects the proximal end portion of the main body portion 40a of the sheath 40 and the distal end portion of the outer tube 61. The proximal side connector 63 is provided at the proximal end portion of the outer tube 61, and accommodates the inner tube 51 in the outer tube 61.

The drive shaft 20 and the signal line 30 described above extend from the first hollow portion 41a of the main body portion 40a of the sheath 40 to the hub 52 located at the proximal end portion of the inner tube member 50 through the inside of the outer tube member 60 connected to the proximal side of the main body portion 40a and the inside of the inner tube member 50 a portion of which is inserted into the outer tube member 60.

The imaging core portion 10 described above is integrally coupled to the inner tube member 50 via the drive shaft 20. Therefore, when the inner tube member 50 is pushed in the insertion direction A1, the inner tube member 50 is pushed into the outer tube member 60 in the insertion direction A1. When the inner tube member 50 is pushed into the outer tube member 60 in the insertion direction A1, the imaging core portion 10 coupled to the inner tube member 50 via the drive shaft 20 moves in the main body portion 40a of the sheath 40 in the insertion direction A1. Conversely, when the inner tube member 50 is pulled in the removal direction A2, the inner tube member 50 is pulled out in the removal direction A2 from the inside of the outer tube member 60. When the inner tube member 50 is pulled out in the removal direction A2 from the inside of the outer tube member 60, the imaging core portion 10 coupled to the inner tube member 50 via the drive shaft 20 moves in the removal direction A2 inside the main body portion 40a of the sheath 40.

A connector portion mechanically and electrically connected to the diagnostic imaging apparatus 120 is provided at a proximal end of the hub 52 of the inner tube member 50. That is, the diagnostic imaging catheter 1 is mechanically and electrically connected to the diagnostic imaging apparatus 120 by the connector portion provided on the hub 52 of the inner tube member 50. More specifically, the electric signal line as the signal line 30 of the diagnostic imaging catheter 1 extends from the ultrasound transducer 11a to the connector portion of the hub 52. The electric signal line as the signal line 30 electrically connects the ultrasound transducer 11a and the diagnostic imaging apparatus 120 in a state where the connector portion of the hub 52 is connected to the diagnostic imaging apparatus 120. A reception signal in the ultrasound transducer 11a is transmitted to the diagnostic imaging apparatus 120 via the connector portion of the hub 52, subjected to predetermined processing, and displayed as an image.

### <Diagnostic Imaging Apparatus 120>

As shown in Fig. 1, the diagnostic imaging apparatus 120 includes the motor 121, which is the power source for rotating the drive shaft 20, and a motor 122, which is a power source for moving the drive shaft 20 in the longitudinal direction A. A rotational movement of the motor 122 is converted into an axial movement by a ball screw 123 connected to the motor 122.

More specifically, the diagnostic imaging apparatus 120 of the present embodiment includes the drive unit 120a, a control device 120b, and a monitor 120c. The control device 120b is electrically connected to the drive unit 120a by wire or wirelessly. The monitor 120c can display an image generated by the control device 120b based on a reception signal received from the diagnostic imaging catheter 1. The motor 121, the motor 122, and the ball screw 123 described above of the present embodiment are provided in the drive unit 120a. An operation of the drive unit 120a is controlled by the control device 120b. The control device 120b may be a processor including a CPU and a memory.

The diagnostic imaging apparatus 120 is not limited to the configuration shown in the present embodiment, and may further include, for example, an external input unit such as a keyboard.

Fig. 6 is an in-use state diagram showing a state in which the diagnostic imaging catheter 1 of the present embodiment is inserted into a blood vessel BV. Hereinafter, a feature of the housing 12 of the imaging core portion 10 of the diagnostic imaging catheter 1 will be described with reference to Figs. 2 to 6.

As shown in Fig. 6, the sheath 40 of the diagnostic imaging catheter 1 is inserted into the living body and used. As described above, the imaging core portion 10 and the drive shaft 20 are accommodated in the sheath 40, and the imaging core portion 10 and the drive shaft 20 are inserted into the living body together with the sheath 40 and used.

Here, as shown in Figs. 2 to 6, the side surface of the housing 12 that faces the radial direction B of the sheath 40 includes the inclined portion 71 that is inclined to approach the central axis line O of the drive shaft 20 toward the distal side until reaching the distal end. The inclined portion 71 extends from the proximal side of a distal end 11a2 of the ultrasound transducer 11a as the transmission and reception member 11 to the distal side of the distal end 11a2 of the ultrasound transducer 11a as the transmission and reception member 11.

By providing such an inclined portion 71 on the side surface of the housing 12, it is possible to improve followability to a blood vessel shape. Therefore, even in a bent blood vessel shape as shown in Fig. 6, since the housing 12 of the present embodiment includes the inclined portion 71 on the side surface, the housing 12 is easily brought into surface contact with an inner surface of the sheath 40 extending along the bent blood vessel shape. As a result, the housing 12 moves easily forward and backward along an inner wall of the blood vessel BV (see Fig. 6).

More specifically, the housing 12 of the present embodiment includes the main body portion 12b, the distal end portion 12c, and the proximal end portion 12d. A substantially elliptical columnar recess is formed in the main body portion 12b. The ultrasound transducer 11a as the transmission and reception member 11 is held by the housing 12 with the back surface side on which the support member 15 is disposed accommodated in the recess and the ultrasound transducer 11a being supported on a bottom surface of the recess. Therefore, the support surface 12a of the transmission and reception member 11 of the housing 12 of the present embodiment is the bottom surface of the recess.

The ultrasound transducer 11a of the present embodiment is bonded to the housing 12 by an adhesive or the like in a state of being accommodated in the recess. As shown in Fig. 3, a through-hole 12b1 penetrating to the outside is formed in the vicinity of the bottom surface of the recess formed in the main body portion 12b of the housing 12. An excess adhesive that adheres the ultrasound transducer 11a and the housing 12 leaks to the outside through the through-hole 12b1. Therefore, it is possible to prevent the adhesive from leaking from a side wall of the recess to the front surface side of the ultrasound transducer 11a. Therefore, it is possible to prevent the adhesive from adhering to the ultrasound transmission and reception surface 11a1 of the ultrasound transducer 11a.

As shown in Fig. 4, in an upper side view of the housing 12 as viewed from a support surface 12a side, the inclined portion 71 is formed on side surfaces of the housing 12 located on both sides of the transmission and reception member 11. By providing such an inclined portion 71, the followability of the housing 12 to the blood vessel shape can be improved. The upper side view of the housing 12 as viewed from the support surface 12a side (see Fig. 4) means a side view of the housing 12 as viewed from the support surface 12a side in the radial direction B of the sheath 40. Hereinafter, the upper side view of the housing 12 as viewed from the support surface 12a side will be simply referred to as "the upper side view of the housing 12".

In the present embodiment, in the upper side view of the housing 12 (see Fig. 4), the inclined portion 71 is formed on the side surfaces of the housing 12 located on the both sides of the transmission and reception member 11. However, the present disclosure is not limited to this configuration. In the upper side view (see Fig. 4) of the housing 12, the inclined portion 71 may be formed on the side surface of the housing 12 located at least on one side of the transmission and reception member 11. However, from a viewpoint of improving the followability of the housing 12 to the blood vessel shape, as in the present embodiment, it is preferable that the inclined portion 71 is formed on the side surfaces of the housing 12 located on the both sides of the transmission and reception member 11 in the upper side view (see Fig. 4) of the housing 12.

Further, in the present embodiment, the inclined portion 71 is also formed at a position of the side surface of the housing 12 on a back side of the support surface 12a. Specifically, in a horizontal side view of the housing 12 shown in Fig. 3, the inclined portion 71 is formed at the position of the side surface of the housing 12 on the back side of the support surface 12a. By providing such an inclined portion 71, the followability of the housing 12 to the blood vessel shape can be improved. The horizontal side view (see Fig. 3) of the housing 12 means a side view as viewed from a viewpoint in which the support surface 12a of the housing 12 appears linear in the radial direction B of the sheath 40. In the present embodiment, as shown in Fig. 3, in the horizontal side view of the housing 12, not only the support surface 12a but also the ultrasound transmission and reception surface 11a1 of the ultrasound transducer 11a as the transmission and reception member 11 is also linearly seen.

As described above, the inclined portion 71 is provided on the side surface of the housing 12 of the present embodiment at positions on both sides of the ultrasound transducer 11a as the transmission and reception member 11 in the upper side view (see Fig. 4) of the housing 12. Further, on the side surface of the housing 12 of the present embodiment, the inclined portion 71 is provided at a position on the back side of the support surface 12a in the horizontal side view (see Fig. 3) of the housing 12.

Further, in the present embodiment, the inclined portion 71 is formed in the whole circumferential region from the position on one side of the side surface of the housing 12 on which the ultrasound transducer 11a is interposed in the upper side view (see Fig. 4) of the housing 12 to the position on the other side on which the ultrasound transducer 11a is interposed in the upper side view (see Fig. 4) of the housing 12 through the position on the back side of the support surface 12a in the horizontal side view (see Fig. 3) of the housing 12. In this way, the followability of the housing 12 to the blood vessel shape can be further improved.

An angle of the inclined portion 71 with respect to the central axis line O of the drive shaft 20 is preferably increased toward the distal side in the side view of the housing 12 (see Figs. 3 and 4). Specifically, in the side views shown in Figs. 3 and 4, the inclined portion 71 of the present embodiment includes a proximal inclined portion 71a and a distal inclined portion 71b in which the angle of the inclined portion 71 with respect to the central axis line O of the drive shaft 20 is larger than that of the proximal inclined portion 71a. The proximal inclined portion 71a extends substantially linearly in the side views of the housing 12 (see Figs. 3 and 4). The proximal inclined portion 71a extends over the proximal side and the distal side of the distal end 11a2 of the transmission and reception member 11. The distal inclined portion 71b is convexly curved and extends in the side view (see Figs. 3 and 4) of the housing 12. The distal inclined portion 71b is continuous with the distal end of the proximal inclined portion 71a and extends to a distal end surface 12e of the housing 12.

The distal end surface 12e of the housing 12 is a flat surface orthogonal to the central axis line O of the drive shaft 20, but the present disclosure is not limited to this, and for example, may be a curved surface that projects convexly to the distal side. Further, the inclined portion 71 on the side surface of the housing 12 includes by the proximal inclined portion 71a linearly extending and the distal inclined portion 71b convexly curved in the side views (see Figs. 3 and 4). However, the present disclosure is not limited to this configuration. For example, in the side views (see Figs. 3 and 4), the inclined portion may be curved and extend such that the angle of the drive shaft 20 with respect to the central axis line O gradually increases toward the distal side.

Further, the inclined portion 71 of the present embodiment is formed from the main body portion 12b of the housing 12 to the distal end portion 12c. More specifically, the inclined portion 71 of the present embodiment is formed only on the distal side of a proximal end 11a3 of the transmission and reception member 11, and is not formed on the proximal side from the proximal end 11a3 of the transmission and reception member 11. That is, the inclined portion 71 on the side surface of the housing 12 of the present embodiment is not formed on the proximal end portion 12d of the housing 12. However, the inclined portion may extend from the distal end of the housing 12 to the proximal side of the proximal end 11a3 of the transmission and reception member 11.

In the side surface of the housing 12 of the present embodiment, the back side of the support surface 12a is defined by a peripheral surface. More specifically, in the side surface of the housing 12 of the present embodiment, the back side of the support surface 12a is defined by the peripheral surface along an inner peripheral surface of the sheath 40. Therefore, the housing 12 is easily brought into surface contact with the inner surface of the sheath 40, the inner surface of the sheath 40 is not easily damaged, the housing 12 is less likely to be caught in the inner surface of the sheath 40, and the followability of the housing 12 to the blood vessel shape can be enhanced. Further, as described above, in the present embodiment, the inclined portion 71 is formed in the whole circumferential region from the position on one side of the side surface of the housing 12 on which the ultrasound transducer 11a is interposed in the upper side view (see Fig. 4) of the housing 12 to the position on the other side on which the ultrasound transducer 11a is interposed in the upper side view (see Fig. 4) of the housing 12 through the position on the back side of the support surface 12a in the horizontal side view (see Fig. 3) of the housing 12. That is, in the side surface of the housing 12 of the present embodiment, the back side of the support surface 12a is the peripheral surface, on which the inclined portion 71 is formed. Therefore, the followability of the housing 12 to the blood vessel shape can be further improved.

In other words, the distal end portion 12c of the housing 12 of the present embodiment has a substantially truncated conical outer shape. The side surface of the distal end portion 12c is a tapered surface that decreases in diameter from the proximal side toward the distal side. A part of the tapered surface of the distal end portion 12c extends continuously in the main body portion 12b, and the inclined portion 71 is formed on the side surface of the housing 12 by extending to the proximal side of the distal end 11a2 of the ultrasound transducer 11a as the transmission and reception member 11.

In other words, the housing 12 of the present embodiment includes the main body portion 12b including the support surface 12a that supports the transmission and reception member 11, and the distal end portion 12c located on the distal side of the main body portion 12b and including the distal end. The side surface of the housing 12 extends over the main body portion 12b and the distal end portion 12c, and includes a peripheral surface portion formed by the peripheral surface along the inner surface of the sheath 40. The peripheral surface portion is formed by the above inclined portion 71 that approaches the central axis line O of the drive shaft 20 from the proximal side toward the distal side.

Further, as shown in Fig. 3, a proximal protrusion portion 17a protruding toward the inner surface of the sheath 40 from the transmission and reception member 11 supported by the support surface 12a is provided on the proximal side of the side surface of the housing 12 with respect to the support surface 12a. With such a configuration, when the imaging core portion 10 moves forward and backward in the sheath 40, it is possible to prevent the ultrasound transmission and reception surface 11a1 of the ultrasound transducer 11a from abutting against the inner surface of the sheath 40. Therefore, the ultrasound transmission and reception surface 11a1 of the ultrasound transducer 11a is less likely to be damaged.

Further, as shown in Fig. 3, a distal protrusion portion 17b protruding toward the inner surface of the sheath 40 from the transmission and reception member 11 supported by the support surface 12a is provided on the distal side of the side surface of the housing 12 with respect to the support surface 12a. With such a configuration, when the imaging core portion 10 moves forward and backward in the sheath 40, it is possible to prevent the ultrasound transmission and reception surface 11a1 of the ultrasound transducer 11a from abutting against the inner surface of the sheath 40. Therefore, the ultrasound transmission and reception surface 11a1 of the ultrasound transducer 11a is less likely to be damaged.

Further, as shown in Fig. 3, the distal protrusion portion 17b protrudes further toward the inner surface of the sheath 40 than the proximal protrusion portion 17a. With such a configuration, when the imaging core portion 10 moves forward and backward in the sheath 40, it is possible to further prevent the ultrasound transmission and reception surface 11a1 of the ultrasound transducer 11a from abutting against the inner surface of the sheath 40. Therefore, the ultrasound transmission and reception surface 11a1 of the ultrasound transducer 11a is less likely to be damaged.

Further, the distal protrusion portion 17b of the present embodiment is inclined so as to approach the central axis line O of the drive shaft 20 toward the distal side in the horizontal side view (see Fig. 3) of the housing 12. Further, in the horizontal side view (see Fig. 3) of the housing 12, the angle of the distal protrusion portion 17b with respect to the central axis line O of the drive shaft 20 increases toward the distal side, and extends to the distal end surface 12e of the housing 12. By providing such a distal protrusion portion 17b, it is possible to further improve the followability of the housing 12 to the blood vessel shape.

Here, the transmission and reception member 11 of the present embodiment is the ultrasound transducer 11a capable of transmitting and receiving the ultrasound on the ultrasound transmission and reception surface 11a1. A distal end surface 11a4 including the distal end 11a2 of the ultrasound transducer 11a is preferably formed of a convex curved surface. The ultrasound transducer 11a of the present embodiment has an elliptical outer shape in a front view of the ultrasound transmission and reception surface 11a1. However, the present disclosure is not limited to this configuration. For example, the ultrasound transducer may be an ultrasound transducer having an outer shape such as a circular shape in a front view of the ultrasound transmission and reception surface, a substantially quadrangular shape in which only the distal end surface is a convex curved surface in the front view of the ultrasound transmission and reception surface, or the like. However, in consideration of a convergence performance of the ultrasound transducer 11a, the outer shape of the ultrasound transmission and reception surface 11a1 is preferably the elliptical shape or the circular shape, and particularly preferably the circular shape.

When a small piezoelectric element 14 (see Fig. 2) capable of being inserted into the living body is used as in the present embodiment, it is preferable to increase an area of the ultrasound transmission and reception surface 11a1 of the ultrasound transducer 11a to implement a high output of the ultrasound. Therefore, in the upper side view (see Fig. 4) of the housing 12, the side surfaces of the housing 12 on the both sides of the ultrasound transducer 11a as the transmission and reception member 11 are likely to be located close to the inner surface of the sheath 40.

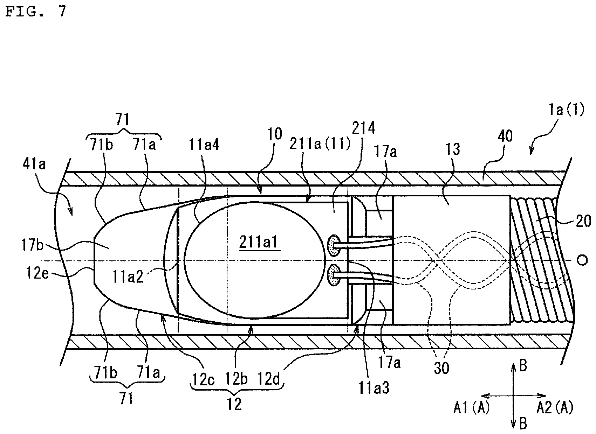

As described above, the distal end surface 11a4 of the ultrasound transducer 11a is formed of the convex curved surface, and the inclined portion 71 is provided along the curved surface of the distal end surface 11a4 of the ultrasound transducer 11a on the side surface of the housing 12 on the both sides of the ultrasound transducer 11a in the upper side view (see Fig. 4) of the housing 12. Therefore, it is possible to further improve the followability of the housing 12 to the blood vessel shape. Further, when the outer shape of the ultrasound transmission and reception surface 11a1 is the elliptical shape or the circular shape, the convergence performance of the ultrasound transducer 11a can also be improved. Fig. 7 is a diagram showing a modification of the ultrasound transducer 11a described above. In an ultrasound transducer 211a shown in Fig. 7, an outer shape of an ultrasound transmission and reception surface 211a1 is an elliptical shape. However, a part of the ultrasound transducer 211a on the proximal side of a piezoelectric element 214 is located at a position not overlapping the ultrasound transmission and reception surface 211a1 in a front view of the ultrasound transmission and reception surface 211a1. A contact portion of the first electrode and the second electrode to which the signal line 30 is electrically connected is provided in the portion not overlapping the ultrasound transmission and reception surface 211a1 in the piezoelectric element 214. According to the ultrasound transducer 211a shown in Fig. 7, the convergence performance of the ultrasound from the ultrasound transmission and reception surface 211a1 can be enhanced as compared with the ultrasound transducer 11a described above.

The diagnostic imaging catheter according to the present disclosure is not limited to the specific configuration specified in the above embodiment, and various modifications and changes can be made without departing from the scope of the claims. The imaging core portion 10 of the above embodiment includes only the ultrasound transducer 11a capable of transmitting and receiving the ultrasonic signal as the transmission and reception member 11. However, the present disclosure is not limited to this configuration. The transmission and reception member 11 may be, for example, an optical element that can transmit and receive the optical signal and that enables optical coherence tomography (abbreviated as "OCT").

### Industrial Applicability

The present disclosure relates to a diagnostic imaging catheter.

### Reference Signs List

1: diagnostic imaging catheter
1a: insertion portion
1b: operation portion
10: imaging core portion
11: transmission and reception member
11a: ultrasound transducer
11a1: ultrasound transmission and reception surface
11a2: distal end of transmission and reception member
11a3: proximal end of transmission and reception member
11a4: distal end surface of ultrasound transducer
12: housing
12a: support surface
12b: main body portion
12b1: through-hole
12c: distal end portion
12d: proximal end portion
12e: distal end surface
13: contrast marker
14: piezoelectric element
15: support member
16: acoustic matching member
17a: proximal protrusion portion
17b: distal protrusion portion
18: absorbing member
20: drive shaft
30: signal line
40: sheath
40a: main body portion
40b: guide wire insertion portion
41a: first hollow portion
41b: second hollow portion
42: marker portion
43: marker portion
44: window portion
46: communication hole
50: inner tube member
51: inner tube
52: hub
60: outer tube member
61: outer tube
62: distal side connector
63: proximal side connector
71: inclined portion
71a: proximal inclined portion
71b: distal inclined portion
100: diagnostic imaging system
120: diagnostic imaging apparatus
120a: drive unit
120b: control device
120c: monitor
121: motor
122: motor
123: ball screw
211a: ultrasound transducer
211a1: ultrasound transmission and reception surface
214: piezoelectric element
A: longitudinal direction of diagnostic imaging catheter
A1: insertion direction of diagnostic imaging catheter
A2: removal direction of diagnostic imaging catheter
B: radial direction of sheath
O: central axis line of drive shaft
W: guide wire
BV: blood vessel

## Claims

1. A diagnostic imaging catheter (1) comprising:
a sheath (40) configured to be inserted into a living body;
a drive shaft (20) rotatable in the sheath (40); and
an imaging core portion (10) attached to the drive shaft (20) in the sheath (40), wherein
the imaging core portion (10) includes:
a transmission and reception member (11) capable of transmitting and receiving a signal; and
a housing (12) holding the transmission and reception member (11),
wherein a side surface of the housing (12) that faces a radial direction (B) of the sheath (40) includes an inclined portion (71) that is inclined to approach a central axis line (O) of the drive shaft (20) toward a distal side until reaching a distal end,
wherein the inclined portion (71) extends from a proximal side of a distal end (11a2) of the transmission and reception member (11) to a distal side of the distal end (11a2) of the transmission and reception member (11),
wherein the transmission and reception member (11) are an ultrasound transducer (11a) capable of transmitting and receiving ultrasound on a ultrasound transmission and reception surface, and
wherein a distal end surface (11a4) of the ultrasound transducer (11a) is formed of a convex curved surface,
**characterized in that** the ultrasound transducer (11a) as the transmission and reception member (11) is held by the housing (12) with a back surface side on which a support member (15) is disposed accommodated in a recess and the ultrasound transducer (11a) is supported on a bottom surface of the recess.

2. The diagnostic imaging catheter (1) according to claim 1, wherein
a side surface of the housing (12) includes a support surface (12a) that supports the transmission and reception member (11), and
in an upper side view of the housing (12) as viewed from a support surface side, the inclined portion (71) is formed on the side surface of the housing (12) located on at least one side of the transmission and reception member (11).

3. The diagnostic imaging catheter (1) according to claim 1, wherein
a side surface of the housing (12) includes a support surface (12a) that supports the transmission and reception member (11), and
the inclined portion (71) is formed at a position of the side surface of the housing (12) on a back side of the support surface (12a).

4. The diagnostic imaging catheter (1) according to claim 2 or 3, wherein
a back side of the support surface (12a) of the side surface of the housing (12) is defined by a peripheral surface.

5. The diagnostic imaging catheter (1) according to any one of claims 2 to 4, wherein
a proximal protrusion portion (17a) protruding toward an inner surface of the sheath (40) from the transmission and reception member (11) supported by the support surface (12a) is provided on a proximal side of the side surface of the housing (12) with respect to the support surface (12a).

6. The diagnostic imaging catheter (1) according to any one of claims 2 to 5, wherein
a distal protrusion portion (17b) protruding toward an inner surface of the sheath (40) from the transmission and reception member (11) supported by the support surface (12a) is provided on a distal side of the side surface of the housing (12) with respect to the support surface (12a).

7. The diagnostic imaging catheter (1) according to any one of claims 1 to 6, wherein
an angle of the inclined portion (71) with respect to the central axis line (O) increases toward the distal side.

## Patentansprüche

1. Diagnostischer Bildgebungskatheter (1), umfassend:
eine Hülse (40), die so konfiguriert ist, dass sie in einen lebenden Körper eingeführt werden kann;
eine Antriebswelle (20), die in der Hülse (40) drehbar ist; und
einen Bildgebungskernabschnitt (10), der an der Antriebswelle (20) in der Hülse (40) befestigt ist, wobei der Bildgebungskernabschnitt (10) umfasst:
ein Sende- und Empfangselement (11), das in der Lage ist, ein Signal zu senden und zu empfangen; und
ein Gehäuse (12), welches das Sende- und Empfangselement (11) hält,
wobei eine Seitenfläche des Gehäuses (12), die einer radialen Richtung (B) der Hülse (40) zugewandt ist, einen geneigten Abschnitt (71) aufweist, der so geneigt ist, dass er sich einer zentralen Achsenlinie (O) der Antriebswelle (20) in Richtung einer distalen Seite zu nähert, bis er ein distales Ende erreicht,
wobei sich der geneigte Abschnitt (71) von einer proximalen Seite eines distalen Endes (11a2) des Sende- und Empfangselements (11) bis zu einer distalen Seite des distalen Endes (11a2) des Sende- und Empfangselements (11) erstreckt,
wobei das Sende- und Empfangselement (11) ein Ultraschallwandler (11a) ist, welcher in der Lage ist, Ultraschall auf einer Ultraschallsende- und -empfangsfläche zu senden und zu empfangen, und
wobei eine distale Endfläche (11a4) des Ultraschallwandlers (11a) aus einer konvex gekrümmten Oberfläche gebildet ist,
**dadurch gekennzeichnet, dass** der Ultraschallwandler (11a) als das Sende- und Empfangselement (11) durch das Gehäuse (12) mit einer Rückflächenseite gehalten wird, auf der ein Stützelement (15) in einer Ausnehmung untergebracht ist, und der Ultraschallwandler (11a) auf einer Bodenfläche der Ausnehmung abgestützt ist.

2. Diagnostischer Bildgebungskatheter (1) nach Anspruch 1, wobei
eine Seitenfläche des Gehäuses (12) eine Stützfläche (12a) aufweist, welche das Sende- und Empfangselement (11) stützt, und
in einer Seitenansicht von oben des Gehäuses (12), von einer Stützflächenseite aus gesehen, der geneigte Abschnitt (71) an der Seitenfläche des Gehäuses (12) ausgebildet ist, die sich auf mindestens einer Seite des Sende- und Empfangselements (11) befindet.

3. Diagnostischer Bildgebungskatheter (1) nach Anspruch 1, wobei
eine Seitenfläche des Gehäuses (12) eine Stützfläche (12a) aufweist, die das Sende- und Empfangselement (11) stützt, und
der geneigte Abschnitt (71) an einer Position der Seitenfläche des Gehäuses (12) auf einer Rückseite der Stützfläche (12a) ausgebildet ist.

4. Diagnostischer Bildgebungskatheter (1) nach Anspruch 2 oder 3, wobei
eine Rückseite der Stützfläche (12a) der Seitenfläche des Gehäuses (12) durch eine Umfangsfläche definiert ist.

5. Diagnostischer Bildgebungskatheter (1) nach einem der Ansprüche 2 bis 4, wobei
ein proximaler Vorsprungsabschnitt (17a), der in Richtung einer Innenfläche der Hülse (40) von dem Sende- und Empfangselement (11), das von der Stützfläche (12a) gestützt wird, vorsteht, an einer proximalen Seite der Seitenfläche des Gehäuses (12) in Bezug auf die Stützfläche (12a) vorgesehen ist.

6. Diagnostischer Bildgebungskatheter (1) nach einem der Ansprüche 2 bis 5, wobei
ein distaler Vorsprungsabschnitt (17b), der in Richtung einer Innenfläche der Hülse (40) von dem Sende- und Empfangselement (11), das von der Stützfläche (12a) gestützt wird, vorsteht, an einer distalen Seite der Seitenfläche des Gehäuses (12) in Bezug auf die Stützfläche (12a) vorgesehen ist.

7. Diagnostischer Bildgebungskatheter (1) nach einem der Ansprüche 1 bis 6, wobei
ein Winkel des geneigten Abschnitts (71) in Bezug auf die zentrale Achsenlinie (O) in Richtung der distalen Seite zunimmt.

## Revendications

1. Cathéter d'imagerie diagnostique (1) comprenant :
une gaine (40) configurée pour être insérée dans un corps vivant ;
un arbre d'entraînement (20) pouvant tourner dans la gaine (40) ; et
une partie centrale d'imagerie (10) fixée à l'arbre d'entraînement (20) dans la gaine (40), dans lequel la partie centrale d'imagerie (10) inclut :
un élément d'émission et de réception (11) apte à émettre et à recevoir un signal ; et
un boîtier (12) contenant l'élément d'émission et de réception (11),
dans lequel
une surface latérale du boîtier (12) qui est tournée dans une direction radiale (B) de la gaine (40) inclut une partie inclinée (71) qui est inclinée pour approcher une ligne d'axe central (O) de l'arbre d'entraînement (20) vers un côté distal jusqu'à atteindre une extrémité distale,
dans lequel
la partie inclinée (71) s'étend d'un côté proximal d'une extrémité distale (11a2) de l'élément d'émission et de réception (11) à un côté distal de l'extrémité distale (11a2) de l'élément d'émission et de réception (11),
dans lequel
l'élément d'émission et de réception (11) est un transducteur à ultrasons (11a) apte à émettre et à recevoir des ultrasons sur une surface d'émission et de réception d'ultrasons, et
dans lequel
une surface d'extrémité distale (11a4) du transducteur à ultrasons (11a) est formée d'une surface incurvée convexe,
**caractérisé en ce que**
le transducteur à ultrasons (11a), en tant qu'élément d'émission et de réception (11), est maintenu par le boîtier (12) avec un côté surface arrière sur lequel un élément de support (15) est disposé logé dans un évidement, et le transducteur à ultrasons (11a) est supporté sur une surface inférieure de l'évidement.

2. Cathéter d'imagerie diagnostique (1) selon la revendication 1, dans lequel
une surface latérale du boîtier (12) inclut une surface de support (12a) qui supporte l'élément d'émission et de réception (11), et
dans une vue latérale supérieure du boîtier (12), tel qu'observé depuis un côté surface de support, la partie inclinée (71) est formée sur la surface latérale du boîtier (12) située sur au moins un côté de l'élément d'émission et de réception (11).

3. Cathéter d'imagerie diagnostique (1) selon la revendication 1, dans lequel
une surface latérale du boîtier (12) inclut une surface de support (12a) qui supporte l'élément d'émission et de réception (11), et
la partie inclinée (71) est formée en une position de la surface latérale du boîtier (12) sur un côté arrière de la surface de support (12a).

4. Cathéter d'imagerie diagnostique (1) selon la revendication 2 ou 3, dans lequel
un côté arrière de la surface de support (12a) de la surface latérale du boîtier (12) est défini par une surface périphérique.

5. Cathéter d'imagerie diagnostique (1) selon l'une quelconque des revendications 2 à 4, dans lequel
une partie de saillie proximale (17a) faisant saillie vers une surface intérieure de la gaine (40) depuis l'élément d'émission et de réception (11) supporté par la surface de support (12a) est disposée sur un côté proximal de la surface latérale du boîtier (12) par rapport à la surface de support (12a).

6. Cathéter d'imagerie diagnostique (1) selon l'une quelconque des revendications 2 à 5, dans lequel
une partie de saillie distale (17b) faisant saillie vers une surface intérieure de la gaine (40) depuis l'élément d'émission et de réception (11) supporté par la surface de support (12a) est disposée sur un côté distal de la surface latérale du boîtier (12) par rapport à la surface de support (12a).

7. Cathéter d'imagerie diagnostique (1) selon l'une quelconque des revendications 1 à 6, dans lequel
un angle de la partie inclinée (71) par rapport à la ligne d'axe central (O) augmente vers le côté distal.
